# EUROPEAN PATENT SPECIFICATION

(11) **EP 3 289 957 B1**
(45) Date of publication and mention of the grant of the patent: **19.07.2023**
(21) Application number: 17184007.7
(22) Date of filing: 31.07.2017
(51) Int. Cl.: A61B 1/00, A61B 1/04, A61B 1/05, A61B 1/06, G02B 23/24, H04N 23/74, H04N 25/531, H04N 25/534, A61B 5/1459, H04N 23/50, A61B 5/1455

(54) **ENDOSCOPE SYSTEM**
ENDOSKOPSYSTEM
SYSTÈME D'ENDOSCOPE

(30) Priority: 31.08.2016 JP 2016169957
(43) Date of publication of application: 07.03.2018
(73) Proprietor: FUJIFILM Corporation, Tokyo 106-8620 (JP)
(72) Inventor: YANO, Takashi, Kanagawa, Kanagawa 258-8538 (JP); OZAWA, Satoshi, Kanagawa, Kanagawa 258-8538 (JP); OTANI, Kenichi, Kanagawa, Kanagawa 258-8538 (JP)
(74) Representative: Klunker IP Patentanwälte PartG mbB

(56) References cited:
- WO-A1-2015/136963
- JP-A- 2016 086 955
- US-A1- 2009 091 614
- US-A1- 2015 022 647
- US-A1- 2016 143 520

## Description

### 1. Field of the Invention

The present invention relates to an endoscope system.

### 2. Description of the Related Art

In the medical field, it is common to perform diagnosis using an endoscope system including a light source device, an endoscope, and a processor device. The light source device generates illumination light. The endoscope images an observation target using an image sensor. The processor device generates an image of the observation target, and displays the image on a monitor.

There are some image sensors capable of adjusting the length of a period (hereinafter, referred to as an accumulation period) that is a maximum period in which light from the observation target can be photoelectrically converted and the electric charges can be accumulated. In a case where the image sensor capable of adjusting the length of the accumulation period or the like is a color sensor, the length of the accumulation period or the like can be adjusted for each color of RGB, for example. For example, in the endoscope system disclosed in JP2016-086955A, the image quality is improved by making the accumulation period of a red pixel that receives red light longer than the accumulation period of a pixel that receives light of other colors.

US 2015/022647 A1 discloses an endoscope system having a light source unit, an image sensor and a light source control unit which changes a wavelength band or a spectrum of the illumination light of each imaging frame imaged by the image sensor. An image processing unit generates one observation image using plural captured image in plural imaging frames having different wavelength bands or different spectra of the illumination light. The imaging control unit keeps constant a total time of the accumulation periods and readout periods of the plural imaging frames. A length of the accumulation period or the readout period for each imaging frame is extended or shortened within the constant total time.
US 2016/143520 A1 discloses and endoscope system in which light components RGB are captured within one frame.
US 2009/09164 A1 discloses an endoscope apparatus having a spectral signal creation part for creating a signal, which corresponds to an optical wavelength narrowband image. A color adjusting unit allocates a different color tone for each of a plurality of bands forming the spectral signal. The image quality input to a display device is adjusted.
WO 2015/136963 A1 discloses an endoscope system in which a subject is alternatingly irradiated with first and second illumination light which have different spectral characteristics. The accumulation time and the readout time inside the total time are not identical for consecutive imaging frames.

### SUMMARY OF THE INVENTION

In a recent endoscope system, there is a case where an image sensor capable of adjusting the accumulation period or the like is mounted. Using the image sensor capable of adjusting the accumulation period or the like is advantageous in that the quality of an image for observation (hereinafter, referred to as an observation image) to be displayed on the monitor can be improved by adjusting the accumulation period or the like as disclosed in JP2016-086955A.

On the other hand, a frame rate (hereinafter, referred to as a display rate) in the case of displaying the observation image on the monitor needs to be equal to or greater than a predetermined value. This is for performing diagnosis or the like without stress while viewing the observation image displayed on the monitor. In a case where the endoscope system has a plurality of observation modes, the display rate may be different for each observation mode. However, the display rate is usually fixed at least for each observation mode in order to secure the display quality or the like.

The image sensor images the observation target in a unit of an imaging frame. Accordingly, in a case where the accumulation period or the like is extended or shortened in the case of using the image sensor capable of adjusting the length of the accumulation period or the like, the length of the imaging frame may change at least partially in the case of continuously imaging the observation target. That is, in the case of arbitrarily extending or shortening the accumulation period or the like just because the accumulation period or the like can be adjusted, the acquisition timing of a captured image changes, and as a result, troubles, such as display delay, may occur in display of the observation image. Even if, for example, the quality of the observation image is improved due to adjustment of the accumulation period or the like, it becomes difficult to observe the observation target if a delay or the like occurs in the display of the observation image.

Therefore, even in a case where the image sensor capable of adjusting the accumulation period or the like is used, in order to be able to perform diagnosis or the like without stress, it is desirable to adjust the accumulation period or the like within a range where the display rate of the observation image can be maintained. In particular, in the case of generating one observation image using a set of captured images (hereinafter, referred to as a plurality of captured images) that are acquired in a plurality of imaging frames, calculations using a plurality of captured images and the like are performed. For this reason, display delay of the observation image or the like is likely to occur.

It is an object of the invention to provide an endoscope system, which maintains the display rate of an observation image while enjoying the advantages of an image sensor capable of adjusting the accumulation period.

An endoscope system of the invention comprises the features of claim 1. Preferred embodiments are defined by the dependent claims.

In the endoscope system of the invention, the total time of the accumulation period and the readout period of a plurality of imaging frames for acquiring a plurality of captured images used in generating the observation image is kept constant, and the length of the accumulation period or the readout period of each imaging frame in the constant total time is extended or shortened. Therefore, it is possible to maintain the display rate of the observation image while enjoying the advantages of the image sensor capable of adjusting the accumulation period or the like.

### BRIEF DESCRIPTION OF THE DRAWINGS

Fig. 1 is an external view of an endoscope system.
Fig. 2 is a block diagram of the endoscope system.
Fig. 3 is a block diagram showing the configuration of a light source unit.
Fig. 4 is an explanatory diagram showing the pixel arrangement of an image sensor.
Fig. 5 is a timing chart showing the relationship between the operation of an image sensor and illumination light in a case where the accumulation period or the like is not adjusted.
Fig. 6 is a timing chart showing the relationship between the operation of an image sensor and illumination light of the invention.
Fig. 7 is a block diagram showing the configuration of a light source unit in a case where an oxygen saturation observation mode is set as a multi-frame observation mode.
Fig. 8 is a graph showing the light absorption coefficients of oxygenated hemoglobin and reduced hemoglobin.
Fig. 9 is a block diagram showing the configuration of an image processing unit in a case where an oxygen saturation observation mode is set as a multi-frame observation mode.
Fig. 10 is a graph showing a correlation between the signal ratio and the oxygen saturation.
Fig. 11 is a block diagram showing the configuration of a light source unit in a case where a specific depth emphasis observation mode is set as a multi-frame observation mode.
Fig. 12 is a block diagram showing the configuration of an image processing unit in a case where a specific depth emphasis observation mode is set as a multi-frame observation mode.
Fig. 13 is a block diagram showing the configuration of a light source unit in a case where a deep layer blood vessel emphasis observation mode is set as a multi-frame observation mode.
Fig. 14 is a block diagram showing the configuration of an image processing unit in a case where a deep layer blood vessel emphasis observation mode is set as a multi-frame observation mode.
Fig. 15 is a block diagram showing the configuration of a light source unit in a case where a narrowband observation mode is set as a multi-frame observation mode.
Fig. 16 is a timing chart showing the relationship between the operation of an image sensor and illumination light in a case where the readout period is shortened.
Fig. 17 is a timing chart showing the relationship between the operation of an image sensor and illumination light in a case where illumination light is also turned on during the readout period in the oxygen saturation observation mode.
Fig. 18 is a block diagram of a processor device corresponding to the invention in a case where illumination light is also turned on during the readout period.
Fig. 19 is a schematic diagram of a capsule endoscope.

### DESCRIPTION OF THE PREFERRED EMBODIMENTS

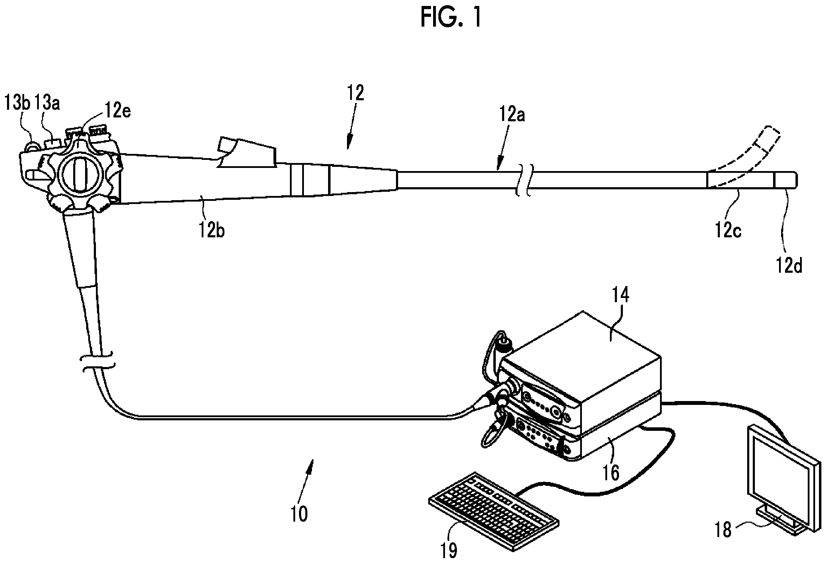

As shown in Fig. 1, an endoscope system 10 has an endoscope 12 for imaging an observation target, a light source device 14 for generating illumination light, a processor device 16 for generating an image for observation (hereinafter, referred to as an observation image) using an image obtained by imaging the observation target (hereinafter, referred to as a captured image), a monitor 18 for displaying the observation image, and a console 19 that is one of user interfaces. The endoscope 12 is optically connected to the light source device 14, and is electrically connected to the processor device 16. The endoscope 12 has an insertion part 12a that is capable of inserted into a subject, an operation unit 12b provided in a proximal end portion of the insertion part 12a, and a bending portion 12c and a distal end portion 12d that are provided on the distal end side of the insertion part 12a. In a case where an angle knob 12e provided in the insertion part 12a is operated, the bending portion 12c is bent. As a result of the bending of the bending portion 12c, the distal end portion 12d faces in a desired direction. An injection port (not shown) for injecting air, water, or the like toward the observation target is provided in the distal end portion 12d.

In addition to the angle knob 12e, a mode selector switch 13a and a zoom operation unit 13b are provided in the operation unit 12b. The mode selector switch 13a is used to switch the observation mode in a case where the endoscope system 10 has a plurality of observation modes. The endoscope system 10 has two kinds of observation modes of a single frame observation mode and a multi-frame observation mode. In the single frame observation mode, an observation image is generated using one or a plurality of captured images acquired in one imaging frame. In the multi-frame observation mode, one observation image is generated using a plurality of captured images acquired in a plurality of imaging frames.

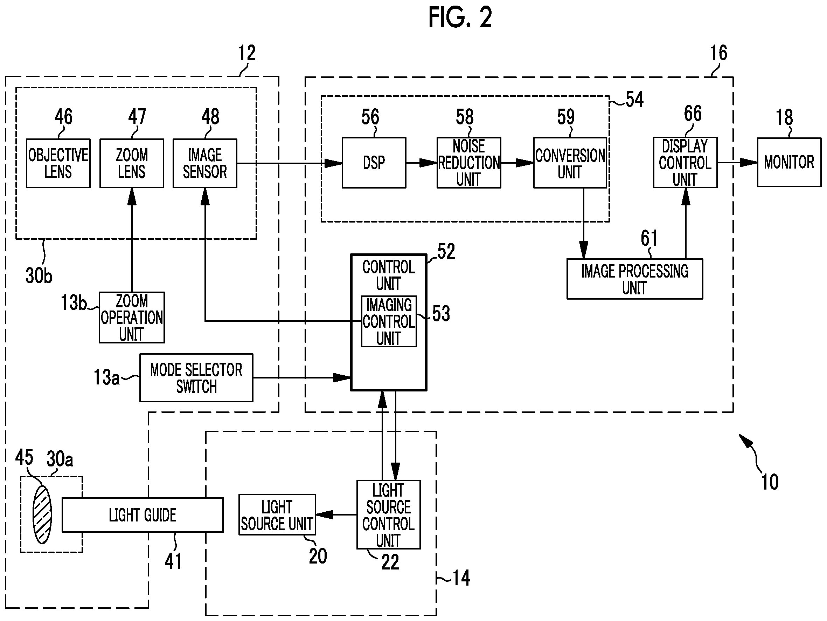

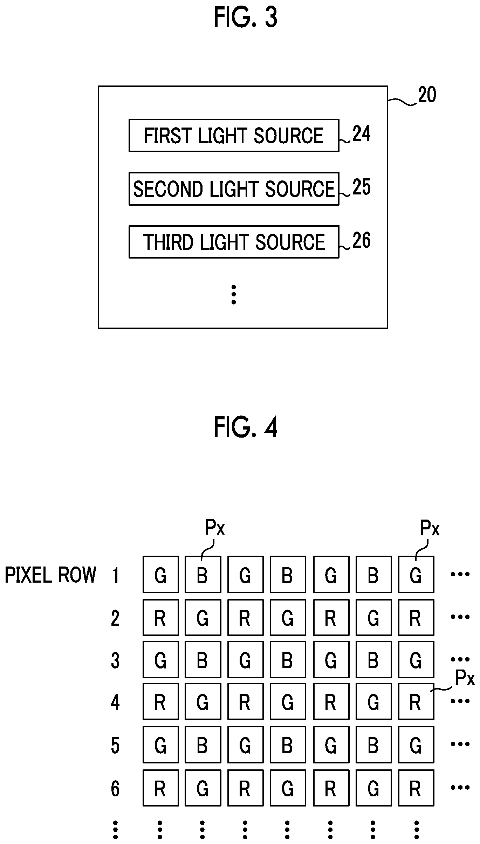

As shown in Fig. 2, the light source device 14 includes a light source unit 20 that generates illumination light and a light source control unit 22 that controls the light source unit 20. As shown in Fig. 3, the light source unit 20 has a plurality of light sources, such as a first light source 24, a second light source 25, and a third light source 26, for example. These light sources can be independently controlled. That is, the first light source 24, the second light source 25, and the third light source 26 can be independently turned on or off. In addition, the first light source 24, the second light source 25, and the third light source 26 can arbitrarily adjust the amount of emitted light (hereinafter, simply referred to as a light amount) in a case where each of the first light source 24, the second light source 25, and the third light source 26 is turned on. The light source unit 20 turns on or off some or all of the plurality of light sources and adjusts the light amount of the light source to be turned on, so that a plurality of kinds of illumination light having different wavelength bands or different spectra are emitted.

The color of illumination light is generally determined by the wavelength band of light components included in the illumination light. Accordingly, the "wavelength band of illumination light is different" means that the color of illumination light is different. In addition, if the spectrum changes even if the wavelength band is the same, that is, if the amount of light component at each wavelength changes, the color of illumination light changes. Accordingly, the "spectrum of illumination light is different" means that the color of illumination light is different. That is, a plurality of kinds of illumination light having different wavelength bands or different spectra are a plurality of kinds of illumination light having different colors.

The first light source 24 is, for example, a blue light source that emits blue light (hereinafter, referred to as B light). The blue color refers to the color of light having a wavelength mainly belonging to a wavelength band of about 400 nm to about 500 nm. The second light source 25 is, for example, a green light source that emits green light (hereinafter, referred to as G light). The green color refers to the color of light having a wavelength mainly belonging to a wavelength band of about 500 nm to about 600 nm. The third light source 26 is, for example, a red light source that emits red light (hereinafter, referred to as R light). The red color refers to the color of light having a wavelength mainly belonging to a wavelength band of about 600 nm to about 700 nm. In the present embodiment, in the case of the single frame observation mode, the light source unit 20 emits white light including B light, G light, and R light. On the other hand, in the present embodiment, in the case of the multi-frame observation mode, the light source unit 20 emits first light and second light having different wavelength bands or different spectra using one or more of B light, G light, and R light.

Light sources provided in the light source unit 20, such as the first light source 24, the second light source 25, and the third light source 26, are, for example, semiconductor light sources, such as light emitting diodes (LED). Instead of the LED, other semiconductor light sources, such as laser diodes (LD), may be used as light sources provided in the light source unit 20. A combination of a semiconductor light source and a phosphor, which emits light of other colors by using light emitted from the semiconductor light source as excitation light, may be used. A lamp light source, such as a xenon lamp, may also be used in the light source unit 20. In addition, a semiconductor light source, a semiconductor light source and a phosphor, and a lamp light source may be combined with an optical filter, which adjusts the wavelength band or the spectrum, to configure the light source of the light source unit 20. For example, by using an optical filter in combination with a white LED, it is possible to emit a plurality of kinds of illumination light.

The light source control unit 22 controls turning on, turning off, and the light amount of a plurality of light sources provided in the light source unit 20, such as the first light source 24, the second light source 25, and the third light source 26, in accordance with the driving timing of an image sensor 48. In particular, in the case of generating one observation image using a plurality of captured images (that is, the multi-frame observation mode), the light source control unit 22 changes the wavelength band or the spectrum of illumination light for each imaging frame for acquiring a plurality of captured images, which are used to generate the observation image, as a result of the control of each light source. The term "turning on" refers that the light source unit 20 emits light with the amount of light enough to image the observation target in the image sensor 48 (that is, to the extent that the image of the observation target can be seen in the observation image). The term "turning off" refers to not only completely stopping the emission of light but also reducing the light amount to such an extent that the image sensor 48 cannot image the observation target. In a case where each of the light sources provided in the light source unit 20 is a semiconductor light source, the light source control unit 22 controls turning on, turning off, and the light amount of each light source by pulse modulation control.

The illumination light generated by the light source unit 20 is incident on a light guide 41. The light guide 41 is built into the endoscope 12 and a universal cord, and propagates the illumination light to the distal end portion 12d of the endoscope 12. The universal cord is a cord for connecting the endoscope 12 with the light source device 14 and the processor device 16. As the light guide 41, it is possible to use a multi-mode fiber. As an example, it is possible to use a small-diameter fiber cable having a diameter of φ0.3 mm to φ0.5 mm that includes a core with a diameter of 105 µm, a cladding with a diameter of 125 µm, and a protective layer as an outer skin.

An illumination optical system 30a and an imaging optical system 30b are provided in the distal end portion 12d of the endoscope 12. The illumination optical system 30a has an illumination lens 45, and emits illumination light to the observation target through the illumination lens 45. The imaging optical system 30b has an objective lens 46, a zoom lens 47, and an image sensor 48. The image sensor 48 images the observation target using reflected light (including scattered light, fluorescence emitted from the observation target, fluorescence due to medicine administered to the observation target, or the like in addition to the reflected light) of the illumination light that returns from the observation target through the objective lens 46 and the zoom lens 47. The zoom lens 47 is moved by operating the zoom operation unit 13b, thereby enlarging or reducing the observation target imaged by using the image sensor 48.

The image sensor 48 images the observation target using the illumination light in a unit of an imaging frame configured to include an accumulation period, which is a maximum period in which electric charges can be accumulated in a pixel Px (refer to Fig. 4), and a readout period for reading out a signal from the pixel Px in which electric charges are accumulated in the accumulation period. The image sensor 48 is, for example, a complementary metal oxide semiconductor (CMOS) sensor, and can arbitrarily adjust the length of the accumulation period and the length of the readout period. Adjusting the length of the accumulation period or the readout period may cause a change in the length of the imaging frame. In the present embodiment, in the entire accumulation period, electric charges are accumulated in each pixel Px. However, the accumulation period is a maximum period in which electric charges can be accumulated as described above, and electric charges do not necessarily need to be accumulated in the entire accumulation period. For example, in the case of actually imaging the observation target, the length of the optimal time to actually accumulate electric charges changes due to the movement of the observation target or the endoscope 12 (for example, brightness change) or the like. For this reason, in the endoscope system 10, the length of the actual time to accumulate electric charges may be finely adjusted for the accumulation period. Fine adjustment of the length of the actual time to accumulate electric charges is performed by, for example, a so-called electronic shutter function of the image sensor 48 (adjustment of the "length of the time to actually accumulate electric charges" with the maximum as "accumulation period") or pulse emission of each light source (adjustment of the substantial length of the light emission time).

The image sensor 48 is a so-called primary color system color sensor. That is, each pixel of the image sensor 48 has one of a blue color filter that transmits blue light, a green color filter that transmits green light, and a red color filter that transmits red light. The pixel Px having a blue color filter is a B pixel ("B"), the pixel Px having a green color filter is a G pixel ("G"), and the pixel Px having a red color filter is an R pixel ("R"). For example, in a case where the illumination light includes B light emitted from the first light source 24, G light emitted from the second light source 25, and R light emitted from the third light source 26, the image sensor 48 receives reflected light of the B light by the observation target at the B pixel, and receives reflected light of the G light by the observation target at the G pixel, and receives reflected light of the R light by the observation target at the R pixel.

As shown in Fig. 4, the image sensor 48 has an imaging surface in which the pixels Px of the respective colors of RGB are arranged. Specifically, B and G pixels are alternately arranged in odd-numbered pixel rows (set of pixels aligned in the horizontal direction in Fig. 4), and G and R pixels are alternately arranged in even-numbered pixel rows. In addition, in the column direction (vertical direction in Fig. 4), B and G pixels are alternately arranged in pixel columns including B pixels, and G and R pixels are alternately arranged in pixel columns including R pixels.

Since the image sensor 48 is a CMOS sensor, signal reading, signal resetting, and the like can be arbitrarily performed for each pixel. In the present embodiment, however, the image sensor 48 collectively performs operations, such as signal reading and signal resetting, for each pixel row. In addition, the image sensor 48 can perform operations, such as signal reading and signal resetting, by selecting an arbitrary pixel row. In the present embodiment, however, reading and resetting of signals of all pixel rows are performed sequentially in ascending order of row numbers. That is, in the present embodiment, the signal reading method of the image sensor 48 is a so-called progressive method.

In addition, the reading method of the image sensor 48 is a so-called rolling shutter method. That is, each pixel Px of the image sensor 48 becomes in a state, in which electric charges can be accumulated by photoelectric conversion, in a case where electric charges accumulated in each pixel are discarded (reset) by reading the signal of each pixel Px. As described above, since the image sensor 48 performs signal reading and signal resetting sequentially for each pixel row, respective pixel rows have different start and end timings of a period in which electric charges can be accumulated by photoelectric conversion in one imaging.

The processor device 16 has a control unit 52, an image acquisition unit 54, an image processing unit 61, and a display control unit 66.

The control unit 52 is a central processing unit (CPU) that performs overall control of the endoscope system 10, and includes an imaging control unit 53 that controls at least the operation of the image sensor 48. For example, the control unit 52 switches the observation mode by receiving an input of a mode switching signal from the mode selector switch 13a and inputting a control signal to the light source control unit 22, the imaging control unit 53, the image processing unit 61, and the like. In addition, the control unit 52 performs synchronous control of the light emission timing of illumination light in the light source unit 20 and the imaging frame in the image sensor 48. Specifically, as a result of the synchronous control of the control unit 52, the light source control unit 22 emits illumination light from the light source unit 20 in the accumulation period and turns off the illumination light in the readout period. In particular, in the case of the multi-frame observation mode, as a result of the synchronous control of the control unit 52, the light source control unit 22 changes the wavelength band or the spectrum of the illumination light for each of a plurality of imaging frames for acquiring a plurality of captured images used in generating the observation image.

The imaging control unit 53 controls the image sensor 48 separately for the accumulation period and the readout period. The accumulation period is a period in which all the pixels Px of the image sensor 48 receive reflected light of illumination light and accumulate electric charges without reading signals from any pixel rows. The readout period is a period subsequent to the accumulation period, in which a signal is read out from each pixel Px of the image sensor 48. In the present embodiment, the imaging control unit 53 resets the pixel Px by discarding the electric charges accumulated in the pixel Px immediately after the signal reading. That is, in the readout period, signal reading and signal resetting are performed.

In the case of the single frame observation mode, the imaging control unit 53 controls the image sensor 48 so as to alternately repeat the accumulation period and the readout period at predetermined time intervals (for example, every 1/60 seconds). Therefore, in the single frame observation mode, the length of the imaging frame is fixed.

On the other hand, in the case of the multi-frame observation mode, the imaging control unit 53 adjusts either the length of the accumulation period or the length of the readout period, or adjusts both the length of the accumulation period and the length of the readout period. At this time, the imaging control unit 53 keeps constant the total time of the accumulation period and the readout period of a plurality of imaging frames for acquiring a plurality of captured images used in generating the observation image, and extends or shortens the length of the accumulation period or the readout period of each imaging frame in the constant total time.

For example, in the multi-frame observation mode, in a case where a plurality of captured images acquired in N ("N" is a predetermined integer) imaging frames (hereinafter, referred to as N imaging frames; the same for a case where N is a specific numerical value) are used to generate the observation image, assuming that the standard length of one imaging frame is "f' seconds (for example, 2/60 seconds), the standard length of the accumulation period is f/2 seconds (for example, 1/60 seconds), the standard length of the readout period is f/2 seconds (for example, 1/60 seconds), and the total time T_{N} of the accumulation period and the readout period for N imaging frame is N × f seconds (for example, 2N/60 seconds). The imaging control unit 53 adjusts either the length of the accumulation period or the length of the readout period or both the length of the accumulation period and the length of the readout period in each imaging frame under the conditions in which the total time T_{N} is kept constant. For example, as a result of extending the accumulation period and adjusting the readout period to the standard length in one imaging frame among the N imaging frames, even if the length of the imaging frame becomes longer than the standard length "f", the accumulation period or the readout period is shortened in another imaging frame to keep a balance. As a result, the total time T_{N} of the accumulation period and the readout period of the N imaging frames is maintained. "Extend or shorten the length of the accumulation period or the readout period of each imaging frame" in the N imaging frames means that the lengths of N accumulation periods and the lengths of N readout periods in the N imaging frames are balanced as described above o maintain the total time T_{N}.

In a case where the imaging control unit 53 "extends or shortens the length of the accumulation period or the readout period of each imaging frame", the total time T_{N} is constant, and there are accumulation periods having different lengths in a case where a plurality of accumulation periods in the N imaging frames are compared or there are readout periods having different lengths in a case where a plurality of readout periods in the N imaging frames are compared. Accordingly, in a case where the total time T_{N} is constant and there are accumulation periods having different lengths in a case where a plurality of accumulation periods in the N imaging frames are compared or in a case where the total time T_{N} is constant and there are readout periods having different lengths in a case where a plurality of readout periods in the N imaging frames are compared, it can be said that the imaging control unit 53 has extended or shortened the length of the accumulation period or the readout period of each imaging frame included in the N imaging frames.

The image acquisition unit 54 acquires a captured image from the image sensor 48 by receiving the signal read from the image sensor 48 by the imaging control unit 53. More specifically, the image acquisition unit 54 acquires three kinds of captured images, that is, a B image formed by the signal read from the B pixel, a G image formed by the signal read from the G pixel, and an R image formed by the signal read from the R image, for each imaging frame.

The image acquisition unit 54 has a digital signal processor (DSP) 56, a noise reduction unit 58, and a conversion unit 59, and performs various kinds of processing on the acquired captured images using these.

The DSP 56 performs various kinds of processing, such as defect correction processing, offset processing, gain correction processing, linear matrix processing, gamma conversion processing, demosaic processing, and YC conversion processing, on the acquired captured images when necessary.

The defect correction processing is processing for correcting the pixel value of each pixel corresponding to the defective pixel of the image sensor 48. The offset processing is processing for setting an accurate zero level by reducing a dark current component from the captured image subjected to the defect correction processing. The gain correction processing is processing for adjusting the signal level of each captured image by multiplying the captured image subjected to the offset processing by the gain. The linear matrix processing is processing for improving the color reproducibility of the captured image subjected to the offset processing, and the gamma conversion processing is processing for adjusting the brightness or the saturation of the captured image after the linear matrix processing. The demosaic processing (also referred to as isotropic processing or simultaneous processing) is processing for interpolating the pixel values of missing pixels, and is applied to the captured image after the gamma conversion processing. The missing pixel is a pixel having no pixel value because pixels of other colors are arranged in the image sensor 48 for the arrangement of color filters. For example, since the B image is a captured image obtained by imaging the observation target in the B pixel, a pixel at a position corresponding to the G or R pixel of the image sensor 48 has no pixel value. The demosaic processing is for generating the pixel values of pixels at the positions of the G and R pixels of the image sensor 48 by interpolating the B image. The YC conversion processing is processing for converting the image after the demosaic processing into a brightness channel Y, a color difference channel Cb, and a color difference channel Cr.

The noise reduction unit 58 performs noise reduction processing on the brightness channel Y, the color difference channel Cb, and the color difference channel Cr using, for example, a moving average method or a median filter method. The conversion unit 59 reconverts the brightness channel Y, the color difference channel Cb, and the color difference channel Cr after the noise reduction processing into captured images of the respective colors of BGR.

The image processing unit 61 performs, for example, color conversion processing, color emphasis processing, structure emphasis processing, and the like on the captured image subjected to the various kinds of processing described above, thereby generating an observation image of one color. In the case of the single frame observation mode, the image processing unit 61 generates one observation image using one or a plurality of captured images acquired in one imaging frame. In the case of the multi-frame observation mode, the image processing unit 61 generates one observation image using a plurality of captured images acquired in a plurality of imaging frames having different wavelength bands or spectra of illumination light. In the color conversion processing, 3 × 3 matrix processing, gradation conversion processing, three-dimensional look-up table (LUT) processing, and the like are performed on the images of the respective colors of BGR. The color emphasis processing is processing for emphasizing the color of the image, and the structure emphasis processing is processing for emphasizing the tissue or structure of the observation target, such as a blood vessel or a pit pattern, for example. The display control unit 66 acquires observation images generated by the image processing unit 61 in a sequential manner, converts the acquired observation images into a suitable format, and sequentially outputs and displays the converted observation images on the monitor 18. As a result, a doctor or the like can observe the observation target using the observation images.

Hereinafter, the operation of the endoscope system 10 in the case of generating one observation image using a plurality of captured images, which are acquired in two imaging frames of a first imaging frame F1 and a second imaging frame F2 by the image processing unit 61, in the multi-frame observation mode will be described.

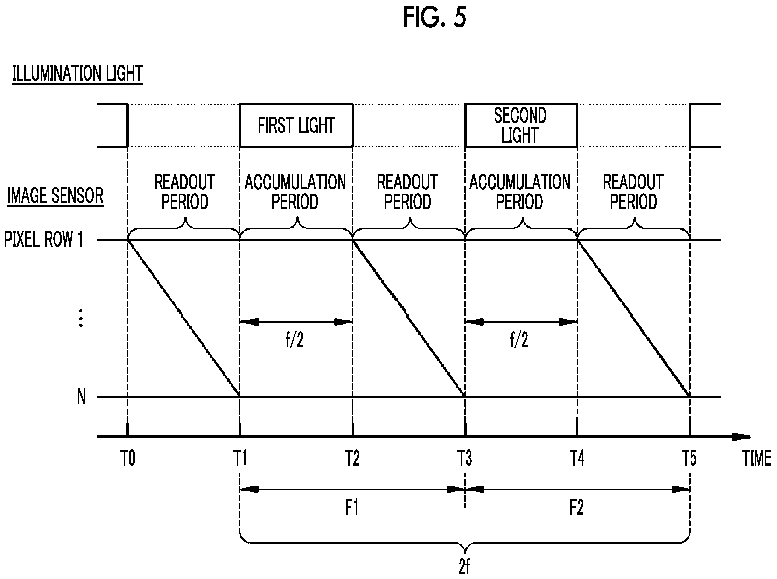

First, as shown in Fig. 5, in a case where the imaging control unit 53 does not adjust the length of the accumulation period or the like of the first imaging frame F1 and the second imaging frame F2, a period of time T1 to time T2 is the accumulation period of the first imaging frame F 1, and a period of time T2 to time T3 is the readout period of the first imaging frame F1. In addition, a period of time T3 to time T4 is the accumulation period of the second imaging frame F2, and a period of time T4 to time T5 is the readout period of the second imaging frame F2. In the present embodiment, for the sake of simplicity, it is assumed that the lengths of the first imaging frame F1 and the second imaging frame F2 are "f" seconds and all of the length of the accumulation period of the first imaging frame F1, the length of the readout period of the first imaging frame F1, the length of the accumulation period of the second imaging frame F2, and the length of the readout period of the second imaging frame F2 are f/2 seconds (1/2 of the length of each imaging frame). Therefore, the total time T₂ of the accumulation period and the readout period of the first imaging frame F1 and the second imaging frame F2 for acquiring a plurality of captured images used in generating one observation image is "2f' seconds.

In addition, as a result of the synchronous control of the control unit 52, the light source control unit 22 emits the first light from the light source unit 20 in the accumulation period of the first imaging frame F1, and emits the second light from the light source unit 20 in the accumulation period of the second imaging frame F2. The first light and the second light are illumination light beams having different wavelength bands or different spectra, which are formed using one or more of B light, G light, and R light. The image acquisition unit 54 acquires a B image, a G image, and an R image in the readout period of the first imaging frame F1, and acquires a B image, a G image, and an R image in the readout period of the second imaging frame F2. Then, the image processing unit 61 generates one observation image using these captured images, and the display control unit 66 displays the observation image generated by the image processing unit 61 on the monitor 18 in a sequential manner.

As described above, in a case where the imaging control unit 53 does not adjust the length of the accumulation period or the like of the first imaging frame F1 and the second imaging frame F2, the image processing unit 61 can generate an observation image at predetermined time intervals equal to the total time T2 (= 2f seconds) of the accumulation period and the readout period of the first imaging frame F1 and the second imaging frame F2 even if there is a lag between the imaging of the observation target and the display of the observation image. As a result, the display control unit 66 can display the observation image on the monitor 18 as a predetermined display rate. However, since the imaging control unit 53 does not adjust the length of the accumulation period or the like of the first imaging frame F1 and the second imaging frame F2 even though the image sensor 48 can adjust the accumulation period or the like, it is not possible to enjoy the advantage of adjusting the length of the accumulation period or the like, such as improving the quality of the observation image.

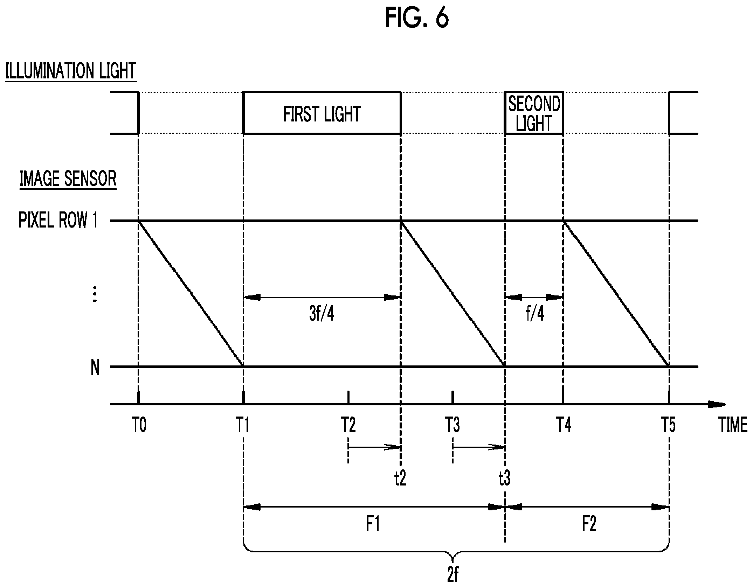

Therefore, in the endoscope system 10, the imaging control unit 53 adjusts the accumulation period or the like of the first imaging frame F1 and the second imaging frame F2. Specifically, as shown in Fig. 6, the imaging control unit 53 delays the end time of the accumulation period of the first imaging frame F1 (start time of the readout period of the first imaging frame F1) from time T2 to time t2. As a result, the accumulation period of the first imaging frame F1 is extended, for example, by f/4 seconds from f/2 seconds of time T1 to time T2 to 3f/4 seconds of time T1 to time t2. At this time, the light source control unit 22 emits the first light using the light source unit 20 in accordance with the extended accumulation period of the first imaging frame F1 under the synchronous control of the control unit 52.

In addition, the imaging control unit 53 delays the end time of the readout period of the first imaging frame F1 from time T3 to time t3. As a result, the imaging control unit 53 maintains the length of the readout period of the first imaging frame F1 at f/2 seconds, which is required to read out all the pixels of the image sensor 48 without insufficiency. Therefore, in the present embodiment, the length from time t2 to time t3 is f/2 seconds.

In a case where the accumulation period of the first imaging frame F1 is extended as described above, the total time of the accumulation period and the readout period of the first imaging frame F1 naturally becomes long as the accumulation period extends. Therefore, in a case where f/2 seconds is secured for each of the accumulation period and the readout period of the second imaging frame F2, the total time T2 of the accumulation period and the readout period of the first imaging frame F1 and the second imaging frame F2 exceeds 2f seconds. In a case where the total time T2 of the accumulation period and the readout period of the first imaging frame F1 and the second imaging frame F2 exceeds 2f seconds, the subsequent processing and the like are delayed. As a result, the timing at which the display control unit 66 displays the observation image on the monitor 18 is also delayed. For this reason, even if the display rate of the observation image is lowered and the quality of the observation image is improved, this may interfere with diagnosis.

Therefore, in a case where the imaging control unit 53 extends the accumulation period of the first imaging frame F1 as described above, the accumulation period of the second imaging frame F2 that starts from time t3 ends at time T4. That is, the imaging control unit 53 shortens the length of the accumulation period of the second imaging frame F2 by f/4 seconds by which the start time of the accumulation period of the second imaging frame F2 has been delayed from time T3 to time t3. Therefore, the readout period of the second imaging frame F2 starts at time T4, and ends at time T5 after f/2 seconds. Needless to say, the light source control unit 22 emits the second light using the light source unit 20 in accordance with the accumulation period of the second imaging frame F2 whose start time has been delayed.

As described above, the imaging control unit 53 shortens the accumulation period of the second imaging frame F2 in a case where the accumulation period of the first imaging frame F1 is extended. As a result, the second imaging frame F2 is shortened by f/4 seconds, by which the accumulation period of the first imaging frame F1 has been extended, and the total time T2 of the accumulation period and the readout period of the first imaging frame F1 and the second imaging frame F2 is kept constant. Therefore, in the multi-frame observation mode of the endoscope system 10, even if the length of the accumulation period or the like is adjusted, no delay occurs in the acquisition of captured images and the subsequent processing and the like in units of two imaging frames of at least the first imaging frame F1 and the second imaging frame F2. For this reason, the display rate of the observation image can be maintained at a display rate that does not interfere with diagnosis or the like.

In addition, the accumulation period of the first imaging frame F1, in which the first light is used as illumination light, is extended. Accordingly, for example, if the first light is illumination light by which a captured image having a good contrast of a tissue or a structure, which is a criterion for diagnosis, can be obtained relative to the second light used in the second imaging frame F2, the visibility of a tissue, a structure, and the like in the observation image is improved. That is, in the endoscope system 10, there are advantages due to adjusting the accumulation period or the like without lowering the display rate of the observation image.

### <Oxygen saturation observation mode>

The endoscope system 10 is suitable for an endoscope system having an observation mode corresponding to the multi-frame observation mode. For example, the endoscope system 10 is particularly suitable for a case having an oxygen saturation observation mode in which the oxygen saturation of the observation target is calculated and an observation image showing the calculated oxygen saturation (hereinafter, referred to as an oxygen saturation image) is generated and displayed.

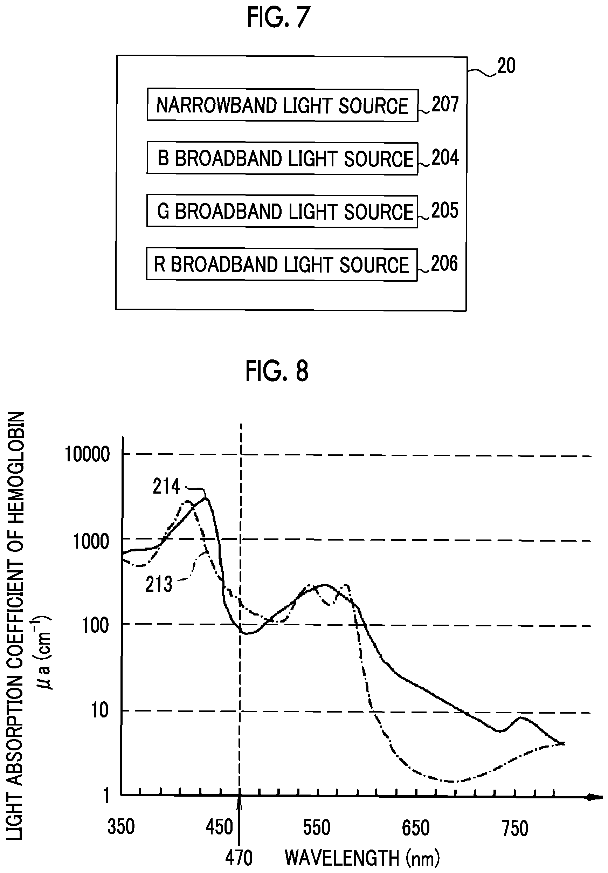

In a case where the oxygen saturation observation mode is set as the multi-frame observation mode, as shown in Fig. 7, a B broadband light source 204, a G broadband light source 205, an R broadband light source 206, and a narrowband light source 207 are provided in the light source unit 20. The B broadband light source 204 is a light source that emits blue broadband light (hereinafter, referred to as B broadband light). Broadband refers to a wavelength band that is wide enough not to be regarded as a single wavelength in the endoscope system 10. For example, a wavelength band of several tens of nm to 100 nm or more is broadband. The G broadband light source 205 is a light source that emits green broadband light (hereinafter, referred to as G broadband light). The R broadband light source 206 is a light source that emits broadband red light (hereinafter, referred to as R broadband light).

The narrowband light source 207 emits narrowband light (hereinafter, referred to as measurement light) for measuring the oxygen saturation. Narrowband refers to a wavelength band that is narrow enough to be regarded as substantially a single wavelength in the endoscope system 10. For example, a wavelength band of ± several tens of nm with respect to the center wavelength is narrowband. The measurement light is a narrowband light having a wavelength, at which the difference between the light absorption coefficients of oxygenated hemoglobin and reduced hemoglobin is large, as a center wavelength. As shown in Fig. 8, for example, at a wavelength of about 470 nm, the difference between the light absorption coefficient 213 of oxygenated hemoglobin and the light absorption coefficient 214 of reduced hemoglobin increases. In the present embodiment, therefore, the narrowband light source 207 is a light source that emits narrowband light having a center wavelength of about 470 nm. As can be seen from Fig. 8, in addition to about 470 nm, there is a wavelength, at which the difference between the light absorption coefficient 213 of oxygenated hemoglobin and the light absorption coefficient 214 of reduced hemoglobin is large, in the purple, blue, or green wavelength band. Therefore, a light source that emits narrowband light having one of these wavelengths as a center wavelength can be used as the narrowband light source 207.

In the oxygen saturation observation mode, one oxygen saturation image is generated using a plurality of captured images acquired in two imaging frames of the first imaging frame F1 and the second imaging frame F2. Specifically in the accumulation period of the first imaging frame F1, the light source control unit 22 turns on the measurement light using the light source unit 20. Accordingly, in a case where the multi-frame observation mode is the oxygen saturation observation mode, the measurement light is the first light. The captured image acquired in the first imaging frame F1 is a B image obtained by imaging the observation target using the reflected light of the measurement light or the like. Hereinafter, for the sake of distinction, the captured image acquired in the first imaging frame F1 is referred to as a B1 image. In the case of acquiring a G image or an R image in the first imaging frame F1, these are referred to as a G1 image and an R1 image, respectively.

On the other hand, in the accumulation period of the second imaging frame F2, the light source control unit 22 turns on white light including B broadband light, G broadband light, and R broadband light using the light source unit 20. Accordingly, in a case where the multi-frame observation mode is the oxygen saturation observation mode, the white light is the second light. Therefore, the first light and the second light are illumination light beams having different wavelength bands and different spectra. In addition, the captured images acquired in the second imaging frame F2 are a B image obtained by imaging the observation target using reflected light of B broadband light or the like, a G image obtained by imaging the observation target using reflected light of G broadband light or the like, and an R image obtained by imaging the observation target using reflected light of R broadband light or the like. Hereinafter, for the sake of distinction, the captured images acquired in the second imaging frame F2 are referred to as a B2 image, a G2 image, and an R2 image, respectively.

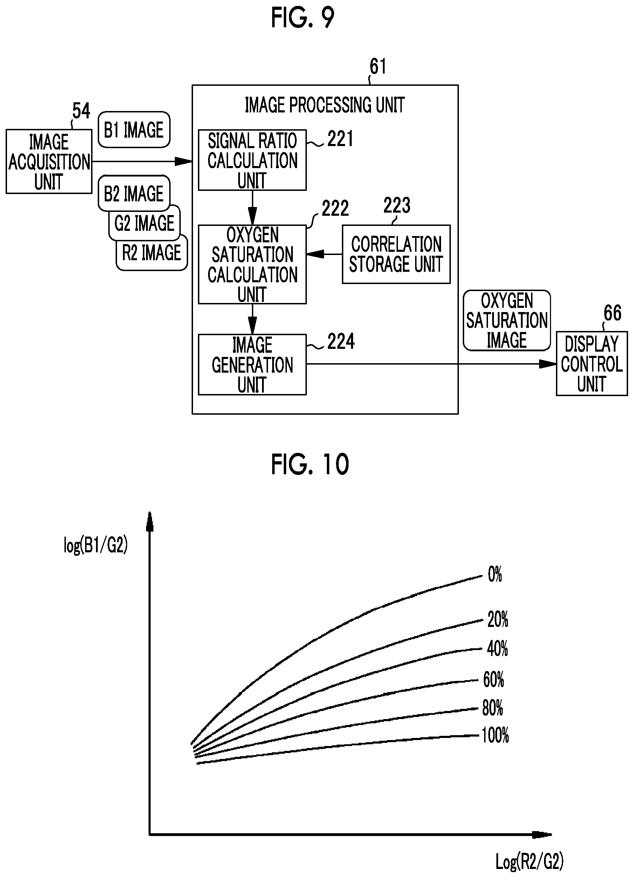

In the oxygen saturation observation mode, an oxygen saturation image is generated using the B1 image, the B2 image, the G2 image, and the R2 image. Therefore, as shown in Fig. 9, a signal ratio calculation unit 221, an oxygen saturation calculation unit 222, a correlation storage unit 223, and an image generation unit 224 are provided in the image processing unit 61.

The signal ratio calculation unit 221 calculates, for example, a ratio of the B1 image to the G2 image (hereinafter, referred to as a signal ratio B1/G2) and a ratio of the G2 image to the R2 image (hereinafter, referred to as a signal ratio R2/G2) for each pixel. The signal ratio B1/G2 changes mainly depending on the value of the oxygen saturation and the blood volume of the observation target, and the signal ratio R2/G2 changes mainly depending on the blood volume of the observation target.

The oxygen saturation calculation unit 222 calculates the oxygen saturation of the observation target for each pixel by comparing the signal ratio B1/G2 and the signal ratio R2/G2 calculated by the signal ratio calculation unit 221 with the correlation stored in the correlation storage unit 223. The correlation storage unit 223 stores the correlation between the signal ratio B1/G2 and the signal ratio R2/G2 and the oxygen saturation on a Log scale, for example, as shown in Fig. 10. In addition, the correlation between the signal ratio B1/G2 and the signal ratio R2/G2 and the oxygen saturation can be calculated in advance by simulation or the like.

The image generation unit 224 generates, for example, an oxygen saturation image that expresses the oxygen saturation of the observation target in colors. Specifically, the image generation unit 224 generates a color observation image using the B2 image, the G2 image, and the R2 image. Thereafter, an oxygen saturation image is generated by coloring each pixel of the generated observation image according to the value of the oxygen saturation calculated by the oxygen saturation calculation unit 222. The image generation unit 224 inputs the generated oxygen saturation image to the display control unit 66, so that the generated oxygen saturation image is displayed on the monitor 18.

In the oxygen saturation observation mode, for example, the imaging control unit 53 extends the accumulation period of the first imaging frame F1 and shortens the accumulation period of the second imaging frame F2 (refer to Fig. 6). For the readout period of the first imaging frame F1 and the readout period of the second imaging frame F2, the imaging control unit 53 adjusts the start or end timing thereof, but maintains the length of each readout period. As a result, the imaging control unit 53 keeps the total time T2 of the accumulation period and the readout period of the first imaging frame F1 and the second imaging frame F2 constant (for example, 2f seconds). Therefore, in the oxygen saturation observation mode, display delay of the oxygen saturation image does not occur. In addition, the imaging control unit 53 improves the brightness, contrast, or the like of the B1 image carrying the information of the oxygen saturation by extending the accumulation period of the first imaging frame F1 in which measurement light is used as illumination light. In a case where the brightness, contrast, or the like of the B1 image is improved, it is possible to calculate the oxygen saturation more accurately than in a case where the length of each of the first imaging frame F1 and the second imaging frame F2 is not adjusted.

### <Specific depth emphasis observation mode>

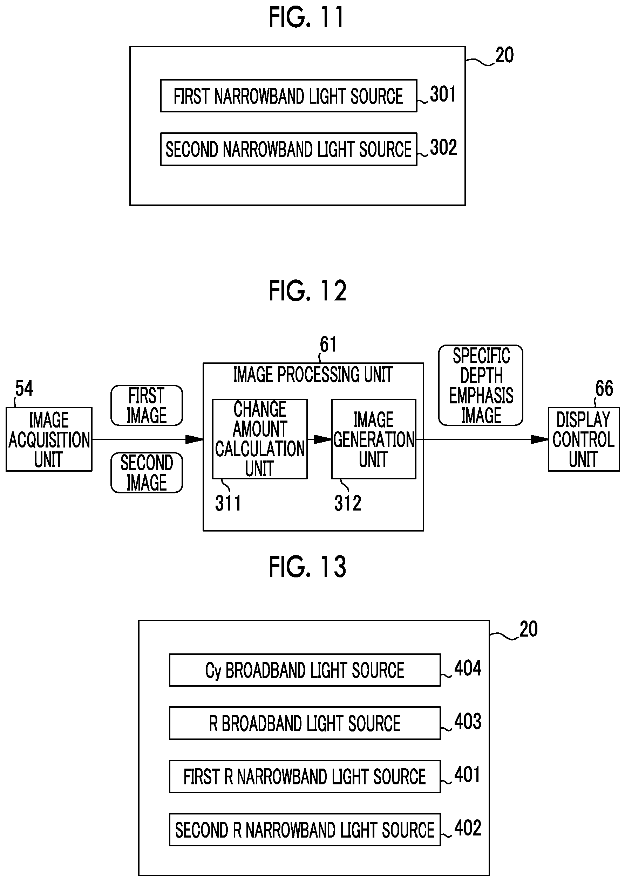

In addition to the oxygen saturation observation mode described above, the invention is also suitable for a specific depth emphasis observation mode for generating and displaying an observation image in which a tissue or a structure located at a specific depth is selectively emphasized (hereinafter, referred to as a specific depth emphasis image), for example. In a case where the specific depth emphasis observation mode is set as the multi-frame observation mode, as shown in Fig. 11, at least a first narrowband light source 301 and a second narrowband light source 302 are provided in the light source unit 20. The first narrowband light source 301 and the second narrowband light source 302 emit narrowband light beams having different center wavelengths. That is, the narrowband light emitted from the first narrowband light source 301 and the narrowband light emitted from the second narrowband light source 302 have different wavelength bands and different spectra. In the present embodiment, the first narrowband light source 301 emits purple narrowband light (hereinafter, referred to as V narrowband light), and the second narrowband light source 302 emits blue narrowband light (hereinafter, referred to as B narrowband light). Purple refers to the color of light mainly having a wavelength of about 380 nm to about 430 nm.

In the specific depth emphasis observation mode, one specific depth emphasis image is generated using a plurality of captured images acquired in two imaging frames of the first imaging frame F1 and the second imaging frame F2. Specifically in the accumulation period of the first imaging frame F1, the light source control unit 22 turns on the V narrowband light using the light source unit 20. Accordingly, in a case where the multi-frame observation mode is the specific depth emphasis observation mode, the V narrowband light is the first light. Since the reflected light of the V narrowband light or the like can be mainly received by the B pixel, the captured image acquired in the first imaging frame F1 is the B1 image. In the accumulation period of the second imaging frame F2, the light source control unit 22 turns on the B narrowband light using the light source unit 20. Accordingly, in a case where the multi-frame observation mode is the specific depth emphasis observation mode, the B narrowband light is the second light. Therefore, the captured image acquired in the second imaging frame F2 is a B2 image obtained by imaging the observation target at the B pixel.

In the specific depth emphasis observation mode, a specific depth emphasis image is generated using the B1 image and the B2 image. Therefore, as shown in Fig. 12, a change amount calculation unit 311 and an image generation unit 312 are provided in the image processing unit 61.

The change amount calculation unit 311 calculates the amount of change between the B1 image and the B2 image for each pixel, and generates a change amount image (not shown) having the amount of change as a pixel value of each pixel. The amount of change is, for example, a ratio or difference between pixels of the B1 image and the B2 image.

The image generation unit 312 generates a specific depth emphasis image by assigning one of the B1 image and the B2 image to a brightness channel and assigning the change amount image generated by the change amount calculation unit 311 to a color difference channel. The specific depth emphasis image is an image emphasizing a tissue, a structure, and the like located at a specific depth determined by the combination of the wavelengths of the V narrowband light and the B narrowband light. The image generation unit 312 inputs the generated specific depth emphasis image to the display control unit 66, so that the generated specific depth emphasis image is displayed on the monitor 18. Since the color difference channel has two channels of a Cr channel and a Cb channel, the image generation unit 312 assigns, for example, change amount images having different weightings to the Cr channel and the Cb channel.

In the specific depth emphasis observation mode, for example, the imaging control unit 53 extends the accumulation period of the first imaging frame F1 and shortens the accumulation period of the second imaging frame F2 (refer to Fig. 6). For the readout period of the first imaging frame F1 and the readout period of the second imaging frame F2, the imaging control unit 53 adjusts the start or end timing thereof, but maintains the length of each readout period. As a result, the imaging control unit 53 keeps the total time T2 of the accumulation period and the readout period of the first imaging frame F1 and the second imaging frame F2 constant (for example, 2f seconds). Therefore, in the specific depth emphasis observation mode, display delay of the specific depth emphasis image does not occur. In addition, the imaging control unit 53 improves the brightness, contrast, or the like of the B1 image by extending the accumulation period of the first imaging frame F1 in which the V narrowband light is used as illumination light. In a case where the brightness, contrast, or the like of the B1 image is improved, it is possible to improve the selection accuracy of "specific depth" to emphasize a tissue, a structure, and the like in the specific depth emphasis image compared with a case where the length of each of the first imaging frame F1 and the second imaging frame F2 is not adjusted.

Needless to say, it may be better to improve the brightness, contrast, or the like of the B2 image depending on the combination of respective specific wavelength bands of the V narrowband light and the B narrowband light. In this case, contrary to the above, the imaging control unit 53 extends the accumulation period of the second imaging frame F2 and shortens the accumulation period of the first imaging frame F1. In the specific depth emphasis observation mode described above, the V narrowband light and the B narrowband light are used. However, in a case where the combination of the wavelength bands of such narrowband light beams is changed, it is possible to change the specific depth to emphasize a tissue, a structure, and the like in the specific depth emphasis image.

### <Deep layer blood vessel emphasis observation mode>

In addition to the oxygen saturation observation mode and the specific depth emphasis observation mode described above, the invention is also suitable for a deep layer blood vessel emphasis observation mode for generating and displaying an observation image in which a thick blood vessel (hereinafter, referred to as a deep layer blood vessel) at a particularly deep position under the mucosa is emphasized (hereinafter, referred to as a deep layer blood vessel emphasis image), for example.

In a case where the deep layer blood vessel emphasis observation mode is set as the multi-frame observation mode, as shown in Fig. 13, for example, a first R narrowband light source 401, a second R narrowband light source 402, an R broadband light source 403, and a Cy broadband light source 404 are provided in the light source unit 20. The first R narrowband light source 401 emits red narrowband light (hereinafter, referred to as first R narrowband light) having a center wavelength of about 600 nm, for example. The second R narrowband light source 402 emits red narrowband light (hereinafter, referred to as second R narrowband light) having a center wavelength of about 630 nm, for example. The R broadband light source 403 emits broadband red light (hereinafter, referred to as R broadband light). The Cy broadband light source 404 emits cyan broadband light (hereinafter, referred to as Cy broadband light) having a wavelength band of about 400 nm to about 550 nm.

In the deep layer blood vessel emphasis observation mode, one deep layer blood vessel emphasis image is generated using a plurality of captured images acquired in two frames of the first imaging frame F1 and the second imaging frame F2. Specifically in the accumulation period of the first imaging frame F1, the light source control unit 22 turns on the first light including the first R narrowband light, the R broadband light, and the Cy broadband light using the light source unit 20. Then, the image acquisition unit 54 acquires a B1 image, a G1 image, and an R1 image as captured images. On the other hand, in the accumulation period of the second imaging frame F2, the light source control unit 22 turns on the second light including the second R narrowband light, the R broadband light, and the Cy broadband light using the light source unit 20. Then, the image acquisition unit 54 acquires a B2 image, a G2 image, and an R2 image as captured images.

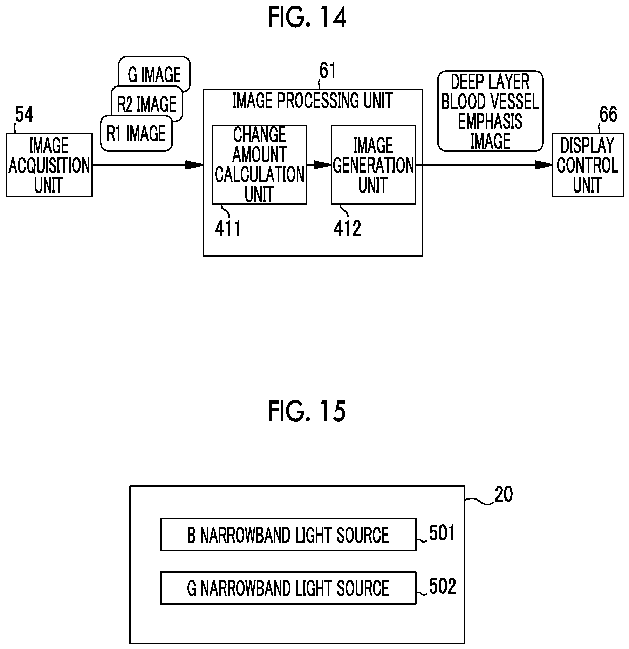

In the deep layer blood vessel emphasis observation mode, a deep layer blood vessel emphasis image is generated using the R1 image and the R2 image and at least one of the G1 image or the G2 image among the captured images described above. Therefore, as shown in Fig. 14, a change amount calculation unit 411 and an image generation unit 412 are provided in the image processing unit 61.

The change amount calculation unit 411 calculates the amount of change between the R1 image and the R2 image for each pixel. The amount of change is, for example, a ratio or difference between pixels of the R1 image and the R2 image. After calculating the amount of change, the change amount calculation unit 411 calculates a correction coefficient based on the calculated amount of change and multiplies the R1 image or the R2 image by the correction coefficient to generate a correction image (not shown). Then, the change amount calculation unit 411 inputs the generated correction image to the image generation unit 412.

The image generation unit 412 generates a deep layer blood vessel emphasis image by assigning one of the R1 image and the R2 image that is not used in generating a correction image, the correction image generated by the change amount calculation unit 411, and the G1 image or the G2 image acquired from the image acquisition unit 54 to a B channel, a G channel, and an R channel, respectively. The image generation unit 412 inputs the generated deep layer blood vessel emphasis image to the display control unit 66, so that the generated deep layer blood vessel emphasis image is displayed on the monitor 18.

In the deep layer blood vessel emphasis observation mode, for example, the imaging control unit 53 extends the accumulation period of the first imaging frame F1 and shortens the accumulation period of the second imaging frame F2 (refer to Fig. 6). For the readout period of the first imaging frame F1 and the readout period of the second imaging frame F2, the imaging control unit 53 adjusts the start or end timing thereof, but maintains the length of each readout period. As a result, the imaging control unit 53 keeps the total time T2 of the accumulation period and the readout period of the first imaging frame F1 and the second imaging frame F2 constant (for example, 2f seconds). Therefore, in the deep layer blood vessel emphasis observation mode, display delay of the deep layer blood vessel emphasis image does not occur. In addition, the imaging control unit 53 improves the brightness, contrast, or the like of the R1 image by extending the accumulation period of the first imaging frame F1 in which the first R narrowband light is included in illumination light. In a case where the brightness, contrast, or the like of the R1 image is improved, it is possible to emphasize a target deep layer blood vessel in the deep layer blood vessel emphasis image more than in a case where the length of each of the first imaging frame F1 and the second imaging frame F2 is not adjusted. Needless to say, it may be better to improve the brightness, contrast, or the like of the R2 image depending on the specific depth of the deep layer blood vessel to be emphasized. In this case, contrary to the above, the imaging control unit 53 extends the accumulation period of the second imaging frame F2 and shortens the accumulation period of the first imaging frame F1.

### < Narrowband observation mode>

In addition to the oxygen saturation observation mode, the specific depth emphasis observation mode, and the deep layer blood vessel emphasis observation mode described above, the invention is also suitable for a narrowband observation mode for generating and displaying a so-called narrowband observation image as an observation image, for example. The narrowband observation image is an observation image in which blood vessels and the like are emphasized by using a captured image obtained by imaging the observation target using blue and green narrowband light.

In a case where the narrowband observation mode is set as the multi-frame observation mode, as shown in Fig. 15, a B narrowband light source 501 and a G narrowband light source 502 are provided in the light source unit 20. The B narrowband light source 501 emits B narrowband light. The B narrowband light has, for example, a center wavelength of about 450 nm, and has a wavelength band of about ± several tens of nm with respect to the center wavelength. The G narrowband light source 502 emits green narrowband light (hereinafter, referred to as G narrowband light). The G narrowband light has, for example, a center wavelength of about 550 nm, and has a wavelength band of about ± several tens of nm with respect to the center wavelength.

In the narrowband observation mode, one narrowband observation image is generated using a plurality of captured images acquired in two imaging frames of the first imaging frame F1 and the second imaging frame F2. Specifically in the accumulation period of the first imaging frame F1, the light source control unit 22 turns on the B narrowband light using the light source unit 20. Accordingly, in a case where the multi-frame observation mode is the narrowband observation mode, the B narrowband light is the first light. The captured image acquired in the first imaging frame F1 is a B1 image. In the accumulation period of the second imaging frame F2, the light source control unit 22 turns on the G narrowband light using the light source unit 20. Accordingly, in a case where the multi-frame observation mode is the narrowband observation mode, the G narrowband light is the second light. The captured image acquired in the second imaging frame F2 is a G2 image. In the narrowband observation mode, the image processing unit 61 generates a narrowband observation image by assigning the B1 image to the B channel and the G channel and assigning the G2 image to the R channel.

In the narrowband observation mode, for example, the imaging control unit 53 extends the accumulation period of the first imaging frame F1 and shortens the accumulation period of the second imaging frame F2 (refer to Fig. 6). For the readout period of the first imaging frame F1 and the readout period of the second imaging frame F2, the imaging control unit 53 adjusts the start or end timing thereof, but maintains the length of each readout period. As a result, the imaging control unit 53 keeps the total time T2 of the accumulation period and the readout period of the first imaging frame F1 and the second imaging frame F2 constant (for example, 2f seconds). Therefore, in the narrowband observation mode, display delay of the narrowband observation image does not occur. In addition, the imaging control unit 53 improves the brightness, contrast, or the like of the B1 image by extending the accumulation period of the first imaging frame F1 in which the B narrowband light is used as illumination light. In a case where the brightness, contrast, or the like of the B1 image is improved, it is possible to more clearly emphasize shallow and narrow blood vessels or the like located near the surface of the mucosa in a narrowband observation image than in a case where the length of each of the first imaging frame F1 and the second imaging frame F2 is not adjusted.

As in each of the observation modes described above, in the case of adjusting the length of the accumulation period or the like in the multi-frame observation mode, the imaging control unit 53 may extend or shorten the length of the accumulation period or the readout period due to the wavelength band, the spectrum, or the amount of illumination light in each imaging frame. For example, in a case where the multi-frame observation mode is the oxygen saturation observation mode, the B1 image obtained by using the measurement light as illumination light mainly carries information of target oxygen saturation. Accordingly, instead of the accumulation period of the second imaging frame F2 in which white light is used as illumination light, the accumulation period of the first imaging frame F1 in which measurement light is used as illumination light is extended. That is, the imaging control unit 53 extends or shortens the length of the accumulation period or the readout period due to the wavelength band or the spectrum of illumination light in each imaging frame. This is the same for other multi-frame observation modes, such as the specific depth emphasis observation mode.

In particular, in the case of adjusting the length of the accumulation period or the like due to the wavelength band of the illumination light used in each imaging frame, it is preferable to increase the accumulation period of the imaging frame in which the wavelength band of the illumination light is narrow. That is, the imaging control unit 53 makes the accumulation period of an imaging frame, in which the wavelength band of the illumination light is relatively narrow, longer than the accumulation period of an imaging frame, in which the wavelength band of the illumination light is relatively wide. The reason is as follows. Firstly, in the case of using the narrowband light as illumination light in the multi-frame observation mode, a captured image acquired in the imaging frame using narrowband light carries information contributing to diagnosis. Secondly, since the amount of light becomes insufficient as the wavelength band becomes narrow, the brightness or the contrast of the captured image tends to be insufficient. For example, in the oxygen saturation observation mode, the measurement light and the white light are used as illumination light. However, the B1 image acquired in the first imaging frame F1 in which measurement light having a relatively narrow wavelength band is used as illumination light has a larger amount of information of target oxygen saturation than the captured image acquired in the second imaging frame F2 has, and the brightness or the contrast of the B1 image acquired in the first imaging frame F1 tends to be insufficient. Therefore, in the oxygen saturation observation mode, the imaging control unit 53 increases the accumulation period of the first imaging frame F1 in which the measurement light is used as illumination light. This is the same for other multi-frame observation modes, such as the specific depth emphasis observation mode.

In addition, in the case of adjusting the length of the accumulation period or the like due to the amount of illumination light used in each imaging frame, it is preferable to increase the accumulation period of the imaging frame in which the amount of illumination light is small. That is, the imaging control unit 53 makes the accumulation period of an imaging frame, in which the amount of illumination light is relatively small, longer than the accumulation period of an imaging frame, in which the amount of illumination light is relatively large. This is because the brightness or the contrast of a captured image acquired in the imaging frame, in which the amount of illumination light is small, tends to decrease. For example, in the oxygen saturation observation mode, the measurement light and the white light are used as illumination light. However, the brightness or the contrast of the B1 image acquired in the first imaging frame F1 in which measurement light having a relatively narrow wavelength band is used as illumination light tends to be lower than the brightness or the contrast of a captured image acquired in the second imaging frame F2. Therefore, in the oxygen saturation observation mode, the imaging control unit 53 increases the accumulation period of the first imaging frame F1 in which the measurement light is used as illumination light. This is the same for other multi-frame observation modes, such as the specific depth emphasis observation mode.

In addition, in the case of adjusting the length of the accumulation period or the like due to the amount of illumination light used in each imaging frame, the imaging control unit 53 may increase the accumulation period of the imaging frame in which illumination light that makes the captured image dark or illumination light that reduces the contrast of the captured image is used. That is, the imaging control unit 53 makes the accumulation period of the imaging frame, in which illumination light that makes the captured image relatively dark or illumination light that reduces the contrast of the captured image relatively is used, longer than the accumulation period of the imaging frame, in which other illumination light that makes the captured image relatively bright or other illumination light that increases the contrast of the captured image relatively is used. Depending on the type of the observation target or the like, illumination light having a specific wavelength band or spectrum may be easily absorbed and the amount of reflected light returning to the image sensor 48 may be small, and accordingly the brightness or the contrast of a captured image that is acquired may be smaller than the levels of other captured images. In such a case, by compensating for the lack of the brightness or the contrast by increasing the accumulation period of the imaging frame, in which illumination light that makes the captured image dark or illumination light that reduces the contrast of the captured image is used, as described above, it is possible to generate and display a clear observation image.

In addition to the above, in the case of adjusting the length of the accumulation period or the like due to the amount of illumination light used in each imaging frame, the imaging control unit 53 may extend the accumulation period of the imaging frame in which the amount of illumination light reaches the maximum amount of light. Usually, in the endoscope system 10, the light source control unit 22 automatically adjusts the amount of illumination light so that the observation target can be observed with an almost constant and appropriate brightness. However, since there is an upper limit to the amount of illumination light due to the performance of each light source provided in the light source unit 20, it is not possible to increase the amount of illumination light exceeding the upper limit. Therefore, in a case where the imaging control unit 53 extends the accumulation period of the imaging frame in which the amount of illumination light reaches the maximum amount of light, it is possible to compensate for the lack of the brightness or the contrast of the captured image acquired in the imaging frame in which the amount of illumination light reaches the maximum amount of light. In this case, the imaging control unit 53 acquires a signal (light amount designation signal) indicating the amount of illumination light from the light source control unit 22 or the control unit 52, and extends the accumulation period using this signal by the amount of light, which is insufficient in a case where the amount of illumination light is set to the maximum amount of light, using the signal.

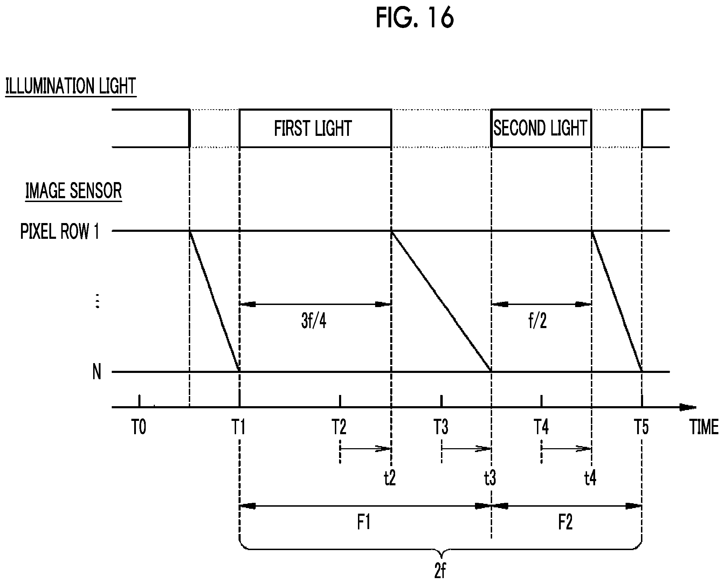

In the multi-frame observation mode of the embodiment described above, the imaging control unit 53 extends or shortens the accumulation period of each imaging frame relatively, but does not change the length of the readout period. However, the imaging control unit 53 can also adjust the length of the readout period. For example, as shown in Fig. 16, in the case of generating one observation image using a plurality of captured images acquired in two imaging frames of the first imaging frame F1 and the second imaging frame F2, the length of the accumulation period of the first imaging frame F1 is extended as in the embodiment described above, and the length of the readout period is maintained. On the other hand, the imaging control unit 53 delays the time T4 of the end of the accumulation period (start of the readout period) to time t4, and maintains the time T5 of the end of the readout period of the second imaging frame F2. As a result, the length of the accumulation period of the second imaging frame F2 is maintained at f/2 seconds, and the length of the readout period of the second imaging frame F2 is shortened to f/4 seconds. In this manner, since the accumulation period of the second imaging frame F2 can be maintained even though the accumulation period of the first imaging frame F1 is extended, it is easy to maintain the brightness or the contrast of the captured image acquired in the second imaging frame F2. Shortening of the readout period can be realized, for example, by so-called partial reading, which is to read out signals from some pixels Px instead of reading out signals from all pixels Px on the imaging surface.

Although the accumulation period of the second imaging frame F2 is maintained in the above-described modification example in which the readout period is shortened, not only the readout period of the first imaging frame F1 but also the accumulation period of the second imaging frame F2 may also be extended. In addition, although the readout period of the first imaging frame F1 is maintained in the modification example described above, the readout period of the first imaging frame F1 can also be shortened. That is, the imaging control unit 53 can arbitrarily combine the extension or shortening of the accumulation period and the shortening of the readout period in the case of adjusting the length of the accumulation period of the image sensor 48 or the like, so that the total time T_{N} of a plurality of imaging frames for acquiring a plurality of captured images used in generating one observation image can be kept constant.

As described above, in the case of shortening the readout period, the imaging control unit 53 may shorten the readout period in an imaging frame different from the imaging frame whose accumulation period is extended. For example, in a case where it is necessary to extend the accumulation period of the first imaging frame F1, the captured image acquired in the first imaging frame F1 carries information greatly contributing to diagnosis in many cases. Therefore, it is better that the length of the readout period of the first imaging frame F1 is maintained as a period (for example, f/2 seconds) in which all the pixels Px can be read. For this reason, in the case of shortening the readout period in order to maintain the total time T_{N}, it is preferable to maintain the length of the readout period of an imaging frame in which the length of the accumulation period is extended and to shorten the readout period of another imaging frame that hardly affects diagnosis.

Needless to say, in the multi-frame observation mode, the imaging control unit 53 can keep the length of one imaging frame constant, and can extend or shorten the lengths of the accumulation period and the readout period in one imaging frame. In this case, since each imaging frame in the multi-frame observation mode can be kept constant, the total time T_{N} can be reliably kept constant. In this case, since the timing at which the image processing unit 61 can acquire a captured image is not changed compared with a case where the accumulation period or the like is not adjusted, display delay of the observation image is particularly unlikely to occur.

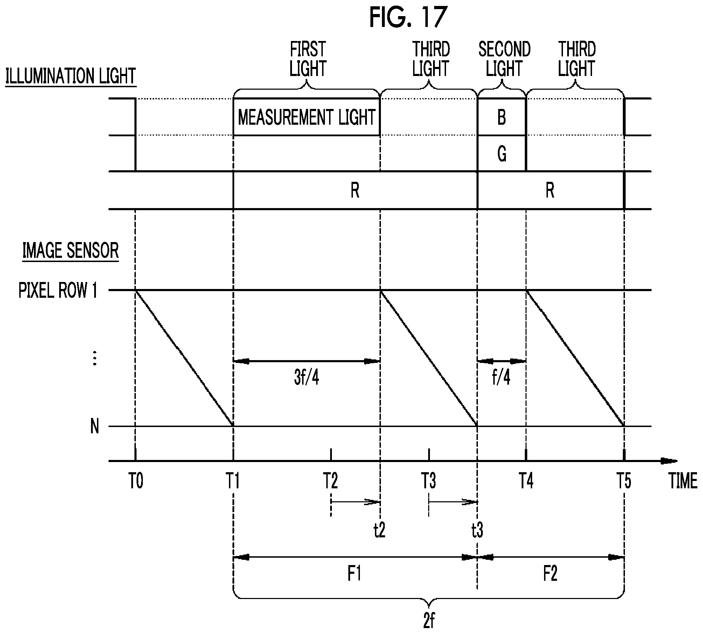

In the embodiment described above, the light source control unit 22 turns on the illumination light in the accumulation period using the light source unit 20, and turns off the illumination light in the readout period. However, the light source control unit 22 may turn on the illumination light in the accumulation period and the readout period. For example, in the case of the oxygen saturation observation mode, as shown in Fig. 17, the light source control unit 22 turns on the first light including the measurement light and the R broadband light (indicated by "R" in Fig. 17) in the accumulation period of the first imaging frame F1, and turns on the third light including the R broadband light in the readout period of the first imaging frame F1. In addition, the light source control unit 22 turns on the second light including the B broadband light (indicated by "B" in Fig. 17), the G broadband light (indicated by "G" in Fig. 17), and the R broadband light in the accumulation period of the second imaging frame F2, and turns on the third light including the R broadband light in the readout period of the second imaging frame F2. That is, in the oxygen saturation observation mode, it is possible to keep the R broadband light on.

As described above, in a case where the R broadband light is turned on in both the accumulation period and the readout period of each imaging frame in the oxygen saturation observation mode, two captured images of the B1 image and the R1 image are obtained in the first imaging frame F1, and three captured images of the B2 image, the G2 image, and the R2 image are obtained in the second imaging frame F2. The oxygen saturation image that is an observation image in the oxygen saturation observation mode can be generated using the B1 image, the B2 image, the G2 image, and the R2 image among these images. Also in the specific depth emphasis observation mode, the deep layer blood vessel emphasis observation mode, and the narrowband observation mode, for example, the R broadband light can be turned on through the accumulation period and the readout period of each imaging frame as in the modification example of the oxygen saturation observation mode described above. In this case, the observation image in each observation mode can be generated in the same manner as in a case where the R broadband light is not turned on in the readout period.

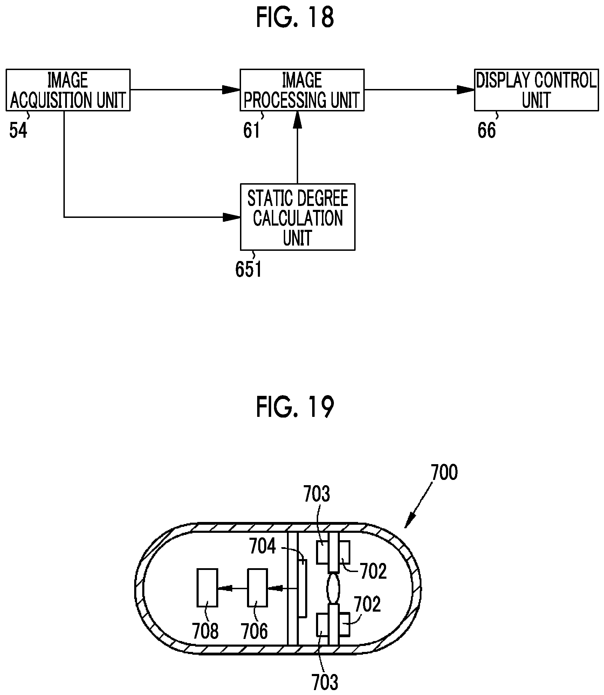

In a case where the illumination light is turned on in the accumulation period and the readout period, as shown in Fig. 18, a stationary degree calculation unit 651 that calculates a stationary degree using a captured image of the color commonly acquired in each imaging frame in the multi-frame observation mode may be provided in the processor device 16. That is, in the case of continuously turning on the R broadband light in the accumulation period and the readout period of the first imaging frame F1 and the second imaging frame F2, the stationary degree calculation unit 651 calculates the stationary degree using either the R1 image and the R2 image or one of the R1 image and the R2 image.

The stationary degree is a numerical value indicating the magnitude of the movement of the observation target, the direction of the movement of the observation target, or the magnitude and direction of the movement of the observation target. The movement of the observation target referred to herein includes not only the movement of the observation target itself but also the relative movement of the observation target due to the movement of the endoscope 12. In the case of calculating the stationary degree using one R1 image or one R2 image, the stationary degree is calculated by frequency analysis, for example. Since it can be evaluated that the movement of the observation target is large if the amount of high frequency components in the R1 image or the R2 image is small, it is possible to calculate the stationary degree based on the amount of high frequency components. In the case of calculating the stationary degree using a plurality of R1 images or a plurality of R2 images, the stationary degree can be calculated from the direction or the magnitude of a motion vector that is calculated from a position between the corresponding points of the observation targets in the R images.

The stationary degree calculated by the stationary degree calculation unit 651 is input to the image processing unit 61, for example. In the image processing unit 61, the stationary degree can be used for registration between the captured image acquired in the first imaging frame F1 and the captured image acquired in the second imaging frame F2. Therefore, in the oxygen saturation observation mode, it is possible to calculate the oxygen saturation more accurately. This is the same for the specific depth emphasis observation mode, the deep layer blood vessel emphasis observation mode, and the narrowband observation mode.

In the embodiment described above, a color sensor is used as the image sensor 48. However, a monochrome sensor in which no color filter is provided in the pixel Px can be used as the image sensor 48. In this case, the B1 image, the G1 image, the R1 image, the B2 image, the G2 image, and the R2 image of the first embodiment can be obtained by imaging the observation target in a sequential manner. For example, six imaging frames are required to acquire all of these six kinds of captured images. That is, in a case where a monochrome sensor is used as the image sensor 48, a larger number of imaging frames than in the first embodiment may be required to generate one observation image, but other than that, the multi-frame observation mode can be set in the same manner as in the embodiment described above.

In the embodiment described above, one observation image is generated using a plurality of captured images acquired in two imaging frames. However, one observation image may be generated using a plurality of captured images acquired in three or more imaging frames. In this case, a larger number of imaging frames than in the first embodiment may be required to generate one observation image, but others excluding this are the same as in the embodiment described above.

In the embodiment described above, the imaging control unit 53 reads out signals from the pixels of the image sensor 48 in a so-called progressive method. However, signals may be read out from the pixels of the image sensor 48 in a so-called interlace method. In this case, the imaging control unit 53 performs signal reading and signal resetting every other pixel row.

In the embodiment described above, the image sensor 48 is a so-called primary color system color sensor. However, it is also possible to use a complementary color system color sensor using a color filter based on a complementary color system, such as cyan, magenta, yellow, and green. A complementary color captured image that is acquired from the complementary color system color sensor can be converted into RGB color images.

In the embodiment described above, the pixel arrangement of the image sensor 48 is a so-called square arrangement. However, the pixel arrangement of the image sensor 48 may be an arrangement other than the square arrangement, such as a so-called honeycomb arrangement.

In the embodiment described above, the light source provided in the light source unit 20 is an LED. However, instead of the LED, other semiconductor light sources, such as laser diodes (LD), may be used in the endoscope system 10. A combination of a semiconductor light source and a phosphor, which emits light of other colors by using light emitted from the semiconductor light source as excitation light, may be used. A lamp light source, such as a xenon lamp, may also be used in the light source unit 20. In addition, a semiconductor light source, a semiconductor light source and a phosphor, and a lamp light source may be combined with an optical filter, which adjusts the wavelength band or the spectrum, to configure the light source of the light source unit 20. For example, by using an optical filter in combination with a white LED, it is possible to generate the light of each color used in the first to fifth embodiments.

In the embodiment described above, the invention is implemented in the endoscope system in that the endoscope 12 including the image sensor 48 is inserted into the subject to observe the inside of the subject. However, the invention is also suitable for a capsule endoscope system. As shown in Fig. 19, for example, the capsule endoscope system includes at least a capsule endoscope 700 and a processor device (not shown).

The capsule endoscope 700 includes a light source unit 702, a control unit 703, an image sensor 704, an image processing unit 706, and a transmitting and receiving antenna 708. The light source unit 702 corresponds to the light source unit 20. The control unit 703 functions similarly to the light source control unit 22 and the control unit 52. The control unit 703 can perform radio communication with the processor device of the capsule endoscope system using the transmitting and receiving antenna 708. Although the processor device of the capsule endoscope system is almost the same as the processor device 16 in each embodiment described above, the image processing unit 706 corresponding to the image acquisition unit 54 and the image processing unit 61 is provided in the capsule endoscope 700, and the observation image is transmitted to the processor device through the transmitting and receiving antenna 708. The image sensor 704 is the same as the image sensor 48.

### Explanation of References

- 10:: endoscope system
- 12:: endoscope
- 12a:: insertion part
- 12b:: operation unit
- 12c:: bending portion
- 12d:: distal end portion
- 12e:: angle knob
- 13a:: mode selector switch
- 13b:: zoom operation unit
- 14:: light source device
- 16:: processor device
- 18:: monitor
- 19:: console
- 20, 702:: light source unit
- 22:: light source control unit
- 30a:: illumination optical system
- 30b:: imaging optical system
- 41:: light guide
- 45:: illumination lens
- 46:: objective lens
- 47:: zoom lens
- 48, 704:: image sensor
- 52, 703:: control unit
- 53:: imaging control unit
- 54:: image acquisition unit
- 56:: DSP (digital signal processor)
- 58:: noise reduction unit
- 59:: conversion unit
- 61, 706:: image processing unit
- 66:: display control unit
- 204:: B broadband light source
- 205:: G broadband light source
- 206, 403:: R broadband light source
- 207:: narrowband light source
- 221:: signal ratio calculation unit
- 222:: oxygen saturation calculation unit
- 223:: correlation storage unit
- 224, 312, 412:: image generation unit
- 301:: first narrowband light source
- 302:: second narrowband light source
- 311, 411:: change amount calculation unit
- 401:: first R narrowband light source
- 402:: second R narrowband light source
- 404:: Cy broadband light source
- 501:: B narrowband light source
- 502:: G narrowband light source
- 651:: stationary degree calculation unit
- 700:: capsule endoscope
- 708:: transmitting and receiving antenna
- f:: length of one imaging frame
- F1:: first imaging frame
- F2:: second imaging frame
- Px:: pixel
- T0 to T5, t2 to t4:: time

## Claims

1. An endoscope system, comprising:
a light source unit (20) adapted to generate illumination light;
an image sensor (48) adapted to image an observation target in a multi-frame observation mode, using the illumination light in a plurality of imaging frames, in which a unit consisting of an accumulation period, which is a maximum period in which electric charges are accumulated in a pixel, and a readout period in which a signal is read out from the pixel is set as a unit of an imaging frame;
a light source control unit (22) adapted to change a wavelength band or a spectrum of the illumination light for each imaging frame while continuously turning on an illumination light having a specific wavelength band or spectrum through the accumulation period and the readout period;
an imaging control unit (53) adapted to read out the signals from the pixels of the image sensor (48) ;
an image processing unit (61) adapted to generate one observation image using a plurality of captured images acquired in a plurality of the imaging frames having different wavelength bands or different spectra of the illumination light;
a stationary degree calculation unit (651) adapted to calculate a stationary degree_of the observation target using the captured image of a color corresponding to the specific wavelength band or the spectrum commonly acquired in each of the imaging frames, wherein the stationary degree of the observation target is a numerical value indicating the magnitude, the direction, or both the magnitude and direction of movement of the observation target relative to the endoscope; wherein
said imaging control unit (53) is adapted to keep constant a total time of the accumulation periods and the readout periods of the plurality of imaging frames for acquiring the plurality of captured images used in generating the one observation image and to extend or to shortens a length of the accumulation period or the readout period of each of the imaging frames in the constant total time,
said image processing unit (61) is adapted to use the stationary degree for registration between the plurality of captured images acquired during the plurality of frames.

2. The endoscope system (10) according to claim 1,
wherein the imaging control unit (53) is adapted to extend or shorten the accumulation period or the readout period of each of the imaging frames due to the wavelength band or the spectrum of the illumination light or an amount of illumination light in each of the imaging frames.

3. The endoscope system (10) according to claim 2,
wherein the imaging control unit (53) is adapted to increase the accumulation period as the wavelength band of the illumination light becomes narrow.

4. The endoscope system (10) according to claim 2 or 3,
wherein the imaging control unit (53) is adapted to increase the accumulation period as the amount of illumination light decreases.

5. The endoscope system (10) according to any one of claims 1 to 4,
wherein the imaging control unit (53) is adapted to increase the accumulation period of the imaging frame in which the illumination light that makes the captured image dark or the illumination light that reduces a contrast of the captured image is used.

6. The endoscope system (10) according to any one of claims 1 to 5,
wherein the imaging control unit (53) is adapted to increase the accumulation period of the imaging frame in which the amount of illumination light reaches a maximum amount of light.

7. The endoscope system (10) according to any one of claims 1 to 6,
wherein the imaging control unit (53) is adapted to shorten the readout period in the imaging frame different from the imaging frame whose accumulation period is extended.

8. The endoscope system (10) according to any one of claims 1 to 7,
wherein the imaging control unit (53) is adapted to keep a length of each one imaging frame constant, and extends or shortens lengths of the accumulation period and the readout period in the one imaging frame.

## Patentansprüche

1. Endoskopsystem, umfassend:
eine Lichtquelleneinheit (20), ausgebildet zum Erzeugen von Beleuchtungslicht;
einen Bildsensor (48), ausgebildet zum Abbilden eines Beobachtungsziels in einem Mehrfachbild-Beobachtungsmodus unter Verwendung des Beleuchtungslichts in einer Mehrzahl von Abbildungs-Einzelbildern, wobei eine Einheit bestehend aus einer Akkumulationszeitspanne, bei der es sich um eine maximale Zeitspanne handelt, in welcher elektrische Ladungen in einem Pixel angesammelt werden, und einer Auslesezeitspanne, in der ein Signal aus dem Pixel ausgelesen wird, als eine Einheit eines Abbildungs-Einzelbilds eingestellt ist;
eine Lichtquellensteuereinheit (22), ausgebildet zum Ändern eines Wellenlängenbands oder eines Spektrums des Beleuchtungslichts für jedes Abbildungs-Einzelbild, während ein Beleuchtungslicht mit einem spezifischen Wellenlängenband oder Spektrum über die Akkumulationszeitspanne und die Auslesezeitspanne kontinuierlich eingeschaltet bleibt;
eine Abbildungssteuereinheit (53), ausgebildet zum Auslesen der Signale aus den Pixeln des Bildsensors (48);
eine Bildverarbeitungseinheit (61), ausgebildet zum Erzeugen eines Beobachtungsbilds unter Verwendung einer Mehrzahl aufgenommener Bilder, die in einer Mehrzahl der Abbildungs-Einzelbilder mit unterschiedlichen Wellenlängenbändern oder unterschiedlichen Spektren des Beleuchtungslichts erfasst werden,
eine Stationärgrad-Berechnungseinheit (651), ausgebildet zum Berechnen eines Stationärgrads des Beobachtungsziels unter Verwendung des aufgenommenen Bilds einer Farbe entsprechend dem spezifischen Wellenlängenband oder dem Spektrum, gemeinsam erfasst in jedem der Abbildungs-Einzelbilder, wobei der Stationärgrad des Beobachtungsziels ein numerischer Wert ist, der den Betrag, die Richtung oder sowohl den Betrag und die Richtung der Bewegung des Betrachtungsziels relativ zu dem Endoskop angibt, wobei
die Abbildungssteuereinheit (53) ausgebildet ist, um eine Gesamtzeit der Akkumulationszeitspannen und der Lesezeitspannen der mehreren Abbildungs-Einzelrahmen zum Erfassen der mehreren aufgenommenen Bilder, die zum Erzeugen des einen Beobachtungsbilds verwendet werden, konstant zu halten, und eine Länge der Akkumulationszeitspanne oder der Auslesezeitspanne jedes der Abbildungs-Einzelbilder in der konstanten Gesamtzeit zu verlängern oder zu verkürzen,
wobei die Bildverarbeitungseinheit (61) ausgebildet ist zum Verwenden des Stationärgrads zur Ausrichtung zwischen den mehreren aufgenommenen Bildern, die während der mehreren Einzelbilder aufgenommen wurden.

2. Endoskopsystem (10) nach Anspruch 1, bei dem die Abbildungssteuereinheit (53) ausgebildet ist zum Verlängern oder Verkürzen der Akkumulationszeitspanne ohne der Auslesezeitspanne für jedes der Abbildungs-Einzelbilder aufgrund des Wellenlängenbands und des Spektrums des Beleuchtungslichts oder einer Menge an Beleuchtungslicht in jedem der Abbildungs-Einzelbilder.

3. Endoskopsystem (10) nach Anspruch 2,
bei dem die Abbildungssteuereinheit (53) ausgebildet ist zum Verlängern der Akkumulationszeitspanne, wenn das Wellenlängenband des Beleuchtungslichts schmal wird.

4. Endoskopsystem (10) nach Anspruch 2 oder 3,
bei dem die Abbildungssteuereinheit (53) ausgebildet ist zum Verlängern der Akkumulationszeitspanne, wenn die Menge des Beleuchtungslichts abnimmt.

5. Endoskopsystem (10) nach einem der Ansprüche 1 bis 4,
bei dem die Abbildungssteuereinheit (53) ausgebildet ist zum Verlängern der Akkumulationszeitspanne des Abbildungs-Einzelbilds, in welchem das Beleuchtungslicht, das das aufgenommene Bild dunkel macht, oder das Beleuchtungslicht, welches einen Kontrast des aufgenommenen Bilds reduziert, verwendet wird.

6. Endoskopsystem (10) nach einem der Ansprüche 1 bis 5,
bei dem die Abbildungssteuereinheit (53) ausgebildet ist zum Erhöhen der Akkumulationszeitspanne des Abbildungs-Einzelbilds, in welchem die Menge des Beleuchtungslichts eine maximale Menge erreicht.

7. Endoskopsystem (10) nach einem der Ansprüche 1 bis 6,
bei dem die Abbildungssteuereinheit (53) ausgebildet ist zum Verkürzen der Auslesezeitspanne in dem Abbildungs-Einzelbild verschieden von dem Abbildungs-Einzelbild, dessen Akkumulationszeitspanne verlängert ist.

8. Endoskopsystem (10) nach einem der Ansprüche 1 bis 7,
bei dem die Abbildungssteuereinheit (53) ausgebildet ist, eine Länge jedes einzelnen Abbildungs-Einzelbild konstant zu halten, und um die Längen der Akkumulationszeitspanne und der Auslesezeitspanne in dem einen Abbildungs-Einzelbild zu verlängern oder zu verkürzen.

## Revendications

1. Système d'endoscope, comprenant :
une unité formant source lumineuse (20) apte à générer une lumière d'éclairage ;
un capteur d'image (48) apte à imager une cible d'observation dans un mode d'observation multi-trame, à l'aide de la lumière d'éclairage dans une pluralité de trames d'imagerie, où une unité consistant en une période d'accumulation, laquelle est une période maximale sur laquelle des charges électriques sont accumulées dans un pixel, et une période de lecture, où un signal est lu à partir du pixel, est réglée comme une unité d'une trame d'imagerie ;
une unité de commande de source lumineuse (22), apte à modifier une bande de longueurs d'ondes ou un spectre de la lumière d'éclairage pour chaque trame d'imagerie tout en mettant sous tension en continu une lumière d'éclairage présentant une bande de longueurs d'onde spécifique ou spectre sur l'ensemble de la période d'accumulation et de la période de lecture ;
une unité de commande d'imagerie (53), apte à lire les signaux à partir des pixels du capteur d'image (48) ;
une unité de traitement d'image (61), apte à générer une image d'observation à l'aide d'une pluralité d'images capturées acquises dans une pluralité de trames d'imagerie présentant des bandes de longueurs d'onde différentes ou des spectres différents de la lumière d'éclairage ;
une unité de calcul de degré stationnaire (651), apte à calculer un degré stationnaire de la cible d'observation à l'aide de l'image capturée d'une couleur correspondant à la bande de longueurs d'ondes spécifique ou au spectre acquis de manière commune dans chacune des trames d'imagerie, dans lequel le degré stationnaire de la cible d'observation est une valeur numérique indiquant l'amplitude, la direction, ou à la fois l'amplitude et la direction de déplacement de la cible d'observation par rapport à l'endoscope ;
dans lequel ladite unité de commande d'imagerie (53) est apte à maintenir constant un temps total des périodes d'accumulation et périodes de lecture de la pluralité de trames d'imagerie pour acquérir la pluralité d'images capturées utilisées lors de la génération de la une image d'observation et rallonger ou raccourcir une longueur de la période d'accumulation ou de la période de lecture de chacune des trames d'imagerie dans le temps total constant, et
ladite unité de traitement d'image (61) est apte à utiliser le degré stationnaire pour un alignement entre la pluralité d'images capturées acquises durant la pluralité de trames.

2. Système d'endoscope (10) selon la revendication 1,
dans lequel l'unité de commande d'imagerie (53) est apte à rallonger ou raccourcir la période d'accumulation ou la période de lecture de chacune des trames d'imagerie dû à la bande de longueurs d'ondes ou au spectre de la lumière d'éclairage ou à une quantité de lumière d'éclairage dans chacune des trames d'imagerie.

3. Système d'endoscope (10) selon la revendication 2,
dans lequel l'unité de commande d'imagerie (53) est apte à augmenter la période d'accumulation à mesure que la bande de longueurs d'onde de la lumière d'éclairage devient étroite.

4. Système d'endoscope (10) selon la revendication 2 ou 3,
dans lequel l'unité de commande d'imagerie (53) est apte à augmenter la période d'accumulation à mesure que la quantité de lumière d'éclairage diminue.

5. Système d'endoscope (10) selon l'une quelconque des revendications 1 à 4,
dans lequel l'unité de commande d'imagerie (53) est apte à augmenter la période d'accumulation de la trame d'imagerie sur laquelle est utilisée la lumière d'éclairage rendant l'image capturée sombre ou la lumière d'éclairage réduisant un contraste de l'image capturée.

6. Système d'endoscope (10) selon l'une quelconque des revendications 1 à 5,
dans lequel l'unité de commande d'imagerie (53) est apte à augmenter la période d'accumulation de la trame d'imagerie sur laquelle la quantité de lumière d'éclairage atteint une quantité maximale de lumière.

7. Système d'endoscope (10) selon l'une quelconque des revendications 1 à 6,
dans lequel l'unité de commande d'imagerie (53) est apte à raccourcir la période de lecture dans la trame d'imagerie différente de la trame d'imagerie dont la période d'accumulation est rallongée.

8. Système d'endoscope (10) selon l'une quelconque des revendications 1 à 7,
dans lequel l'unité de commande d'imagerie (53) est apte à maintenir constante une longueur de chaque une trame d'imagerie, et rallonge ou raccourcit des longueurs de la période d'accumulation et de la période de lecture dans la une trame d'imagerie.
